# EUROPEAN PATENT SPECIFICATION

(11) **EP 4 026 138 B1**
(45) Date of publication and mention of the grant of the patent: **21.05.2025**
(21) Application number: 20758219.8
(22) Date of filing: 20.08.2020
(51) Int. Cl.: G16B 45/00, G16B 10/00, G16H 50/20

(54) **USING NETWORK ANALYSIS AS A TOOL FOR TREATING HUMAN SKIN DYSBIOSIS**
VERWENDUNG VON NETZWERKANALYSE ALS WERKZEUG ZUR BEHANDLUNG VON MENSCHLICHER HAUTDYSBIOSE
UTILISATION DE L'ANALYSE DE RÉSEAU COMME OUTIL DE TRAITEMENT DE LA DYSBIOSE CUTANÉE HUMAINE

(30) Priority: 02.09.2019 WO PCT/CN2019/103965; 15.10.2019 EP 19203373
(43) Date of publication of application: 13.07.2022
(73) Proprietor: Unilever IP Holdings B.V., 3013 AL Rotterdam (NL); Unilever Global IP Limited, Wirral, Merseyside CH62 4ZD (GB)
(72) Inventor: CHU, Chung-Ching, Shanghai, 200335 (CN); PU, Mingming, Shanghai, 200335 (CN); XU, Yining, Shanghai, 200335 (CN)
(74) Representative: James, Helen Sarah
(86) International application number: PCT/EP2020/073270
(87) International publication number: WO 2021/043581

(56) References cited:
- WO-A1-2011/022660
- MARCUSH Y LEUNG ET AL: "Individual and household attributes influence the dynamics of the personal skin microbiota and its association network", MICROBIOME, BIOMED CENTRAL LTD, LONDON, UK, vol. 6, no. 1, 2 February 2018 (2018-02-02), pages 1 - 15, XP021253197, DOI: 10.1186/S40168-018-0412-9
- XINZHAO TONG ET AL: "ABSTRACT", MSYSTEMS, vol. 4, no. 2, 26 March 2019 (2019-03-26), XP055678879, DOI: 10.1128/mSystems.00004-19

## Description

### Field of the invention

The present invention is in the field of human microbiome and particularly in the field of network analysis for treating dysbiotic conditions affecting human skin.

### Background of the invention

Structural and functional characteristics of human microbiome is heavily influenced by microbe-microbe and microbe-host interactions. Understanding interactions amongst microbes and between microbes and hosts is essential for understanding the functions of microbiome. Network theory is an approach for modelling complex biological systems with multifaceted interactions between members, such as microbiota. Complementing to the traditional approach that focuses on individual members of microbes in isolation, network theory provides a holistic approach that focuses on polymicrobial interactions within the microbiome community and interactions with their hosts. Network-based analytical approach allows researchers to model and analyse the complex polymicrobial interactions in a single network.

In a typical association-based network model of a microbial system, network nodes represent taxa and edges represent positive or negative associations between taxa across samples in a defined population. Microbiome network analyses can include description of the architectural features of the microbial community and key topologies of network such as hub nodes, connectors, and modules. The microbiome community assembly is driven by dynamic ecological and evolutionary processes. Applying network theory to microbiome studies may help uncover microbial relationships essential for community assembly or stability, and the potential etiological impact on host. It is known that the level of interaction in a community can predict ecological stability and resilience. Abrupt shifts in abundance of certain microbial genera may be signals of a transition from healthy to diseased state or *vice versa.*

A wide range of methods have been used to construct microbiome networks. The most popular methods are correlation-based techniques to find significant pairwise associations by calculating a correlation coefficient such as Pearson's correlation coefficient or Spearman's nonparametric rank correlation coefficient. However, the limitations of these methods for microbiome data include incorrect correlations due to compositionality and being severely underpowered due to the mass of zero counts. The concerns over correlation-based analyses have led to the development of methods that are robust to compositionality. SparCC (Sparse Correlations for Compositional data) is a methodology that uses linear Pearson's correlations between the log-transformed components to infer associations in compositional data. SPIEC-EASI (SParse InversE Covariance Estimation for Ecological Association Inference) is another statistical method for the inference of microbial ecological networks that combines data transformations developed for compositional data analysis with a graphical model inference framework with the assumption that the underlying ecological association network is sparse.

WO14205088 A1 (Prodermiq) discloses a method of characterizing a microbiome of skin or subcutaneous tissue of a subject. The method includes a) obtaining a sample comprising a plurality of microorganisms from the skin or subcutaneous tissue of the subject; and b) analyzing and classifying the plurality of microorganisms of (a) to characterize the microbiome of the subject, thereby characterizing the microbiome of the subject. In some embodiments, the method further includes comparing the microbiome of the subject to a reference microbiome or generating a microbiome profile of the subject, or identifying a disease or disorder which the subject has, or is at risk of developing, or providing a personalized treatment regime to the subject. In various embodiments, the reference microbiome is classified as having a healthy profile and a similarity between the microbiome of the subject and the reference microbiome identifies the microbiome of the subject as having a healthy profile. Alternatively, the reference microbiome is classified as having, or at risk of having a disease or disorder and a similarity between the microbiome of the subject and the reference microbiome identifies the microbiome of the subject as having as having, or at risk of having the disease or disorder.

NAKATSUJI T. Antimicrobials from human skin commensal bacteria protect against Staphylococcus aureus and are deficient in atopic dermatitis. Sci Transl Med. 2017 February 22; 9(378), discloses that the microbiome can promote or disrupt human health by influencing both adaptive and innate immune functions. The authors tested whether bacteria that normally reside on human skin participate in host defense by killing *S. aureus,* a pathogen commonly found in patients with atopic dermatitis (AD) and an important factor that exacerbates this disease. High-throughput screening for antimicrobial activity against *S*. *aureus* is performed on isolates of coagulase-negative Staphylococcus (CoNS) collected from the skin of healthy and AD subjects. CoNS strains with antimicrobial activity were common on the normal population but rare on AD subjects. A low frequency of strains with antimicrobial activity correlated with colonization by *S*. *aureus.* The antimicrobial activity was identified as previously unknown antimicrobial peptides (AMPs) produced by CoNS species including S. *epidermidis* and *S. hominis.* These AMPs were strain-specific, highly potent, selectively killed *S. aureus,* and synergized with the human AMP LL-37. Application of these CoNS strains to mice confirmed their defense function *in vivo* relative to application of nonactive strains. Strikingly, reintroduction of antimicrobial CoNS strains to human subjects with AD decreased colonization by S. aureus These findings show how commensal skin bacteria protect against pathogens and demonstrate how dysbiosis of the skin microbiome can lead to disease.

US2018311144 A (NATURA COSMETICOS S A) discloses a probiotic cosmetic formulation for the cosmetic treatment of the skin, comprising a microbiota and preferably, betaines and/or prebiotic agents.

WO11022660 A1 (Vedanta Biosciences) discloses methods of diagnosing and treating microbiome-associated diseases or improving health using interaction network parameters to analyze interaction networks between microbes, and between microbes and the host, to determine important (e.g., "highly-connected") organisms or molecules as determined by various network parameters. Methods are provided including and beyond correlation to use these important (e.g., "highly-connected") organisms or molecules as targets for modulation or as therapeutic agents to improve health. The publication also discloses products containing microbiota modulators, probiotics, or other therapeutic agents derived from these important "highly-connected" organisms or molecules for the improvement of health.

This publication considers the connections only in one condition.

However, the present inventors are of the view that following such a method might not help identify all potential target organisms that may be made the subject of an intervention. The changes occurring in network parameters between a dysbiotic and a non-dysbiotic condition could help identify some more potential targets for intervention.

### Summary of the invention

In accordance with this invention is disclosed a method of determining a bacterial strain suitable for treating a human skin dysbiotic condition as defined in claims 1-9, comprising a step of doing network analysis by a computer to determine whether said bacterial strain forms connectivity with at least a second bacterial strain in a dysbiotic condition as well as a non-dysbiotic condition, wherein there is difference in said connectivity between said dysbiotic condition and said non-dysbiotic condition wherein said connectivity means a positive correlation or negative correlation and further wherein said network is generated by co-occurrence analysis of abundance of said bacterial strain and said second bacterial strain by DNA sequencing by following 16s rRNA amplicon or whole genome sequencing method.

### Detailed description of the invention

As used herein the term "comprising" encompasses the terms "consisting essentially of" and "consisting of". Where the term "comprising" is used, the listed steps or options need not be exhaustive. Unless otherwise specified, numerical ranges expressed in the format "from x to y" are understood to include x and y. In specifying any range of values or amounts, any particular upper value or amount can be associated with any particular lower value or amount. Except in the examples and comparative experiments, or where otherwise explicitly indicated, all numbers are to be understood as modified by the word "about". All percentages and ratios contained herein are calculated by weight unless otherwise indicated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way. The examples are intended to illustrate the invention and are not intended to limit the invention to those examples *per se.*

The term "microbiota" refers, collectively, to the entirety of microbes found in association with a higher organism, such as a human. Organisms belonging to a human's microbiota may generally be categorized as bacteria, archaea, yeasts, and single-celled eukaryotes, as wells as viruses and various parasites such as Helminths.

The term "microbiome" refers, collectively, to the entirety of microbes, their genetic elements (genomes), and environmental interactions, found in association with a higher organism, such as a human.

The term "commensal" refers to organisms that are normally harmless to a host, and can also establish mutualistic relations with the host. The human body contains about 100 trillion commensal organisms, which have been suggested to outnumber human cells by a factor to 10.

The term "microbial derived component" refers to a component consisting of, emanating from, or produced by members of the microbiota. The component can be, for example, a microbe, a microbial protein, a microbial secretion, or a microbial fraction.

The term "network" refers to a constructed representation of components (host or microbial-derived components) describing the connection of the components by various methods.

The term "node" refers to a terminal point or an intersection point of a graphical representation of a network. It is the abstraction of an element such as an organism, a protein, a gene, a transcript, or a metabolite.

The term "edge" refers to a link between two nodes. A link is the abstraction of a connection between nodes, such as covariance between the nodes.

The term "highly connected organism" refers to a key functional member of the microbiota that has edge connections to a large number of nodes in the network. For example, a bacterial species may perform biotransformations of numerous metabolites, thus plausibly influencing host metabolism and host health.

The term "metagenomics" refers to genomic techniques for the study of communities of microbial organisms directly in their natural environments, without requiring isolation and lab cultivation of individual species.

Network analysis has long appeared in many fields of computational biology and bioinformatics including genomics, proteomics and metabolomics but it has not been widely used in microbial flora research. Network analysis can find possible functional connections between microbial individuals through the interaction between microbial individuals. This method is based on the species abundance used in traditional ecological analysis, and the inter-species interactions are included in the analysis. There have been some studies that have successfully applied network analysis to human microbial population analysis. Traditional network attributes such as network density mostly ignore the information of nodes and edges in the network. In the microbial flora interaction network, each node represents a species or an operation taxonomic unit (OTU), while the edge represents the interaction between two species or OTUs. In the flora interaction network, the negative relationship between the two sides indicates that there is resistance or inhibition between the two, while the positive relationship with the positive relationship indicates that the two are synergistic. These interactions exist in the realization of the function of the flora network. A more important position, and the occurrence and development of diseases will affect the realization of network functions, thus leading to changes in the interaction between species in the network.

Disclosed in accordance with a first aspect of the invention is a method of determining whether a bacterial strain is a probiotic suitable for treating a human skin dysbiotic condition as defined in claims 1-9, comprising a step of doing network analysis to determine whether said bacterial strain forms connectivity with at least a second bacterial strain in a dysbiotic condition as well as a non-dysbiotic condition, wherein said bacterial strain is said to be suitable if there is difference in said connectivity between said dysbiotic condition and said non-dysbiotic condition, wherein the degree of connectivity is lower in said dysbiotic condition and higher in said non dysbiotic condition. It is preferred that difference in degree of connectivity is at least 40%. Further preferably, the connectivity is at least top 5% amongst total identified bacterial strains in non-dysbiotic skin. Yet further preferably the connectivity is at least top 5% amongst total identified bacterial strains in non-dysbiotic skin and that the difference in connectivity between said non-dysbiotic state and said dysbiotic state is at least 40%. Further preferably in this case the dysbiotic condition is acne.

In accordance with the method of the invention, connectivity means either one of positive correlation or negative correlation. Therefore in one aspect of the invention connectivity means a positive correlation. Alternatively, connectivity means negative correlation.

It is particularly preferred that bacterial strain and said second bacterial strain are strains that are found on human skin.

### Network analysis

It is preferred that the network analysis is conducted by Sparse InversE Covariance estimation for SPIEC-EASI version 0.1.2. SPIEC-EASI (SParse InversE Covariance Estimation for Ecological Association Inference) is a statistical method for the inference of microbial ecological networks from amplicon sequencing datasets that addresses both of these issues. SPIEC-EASI combines data transformations developed for compositional data analysis with a graphical model inference framework that assumes the underlying ecological association network is sparse. To reconstruct the network, SPIEC-EASI relies on algorithms for sparse neighborhood and inverse covariance selection.

Alternatively, the analysis can be performed by any other equivalent technique or computer program.

Further it is preferred that Cytoscape (version 3.5.1) is used to visualize OTU networks and calculate the network connectivity. Cytoscape is an open source software platform for visualizing molecular interaction networks. Cytoscape core distribution provides a basic set of features for data integration, analysis, and visualization. Here again, any other alternative software program may be used. It is preferred that the network analysis is set at the level of OTU (Operational Taxonomic Unit), ASV (Action Script Viewer), species or genus. More preferably the network analysis is set at OTU,

Further preferably the network analysis is generated by co-occurrence analysis of abundance of said first and said second bacterial strains by DNA sequencing by way of 16s rRNA amplicon or whole genome sequencing method.

Preferably the bacterial strain is from at least one of the genus Staphylococcus, Streptococcus, Microbacterium, Methyloversatilis, Deinococcus, Moraxella or Acinetobacter.

Similarly, it is preferred that the second bacterial strain is from at least one of the genus Acidovorax, Actinomyces, Bacillus, Chryseobacterium, Corynebacterium, Fusobacterium, Staphylococcus, Streptococcus, Microbacterium, Methylobacterium, Methyloversatilis, Deinococcus, Micrococcus, Moraxella, Neisseria, Paracoccus, Prevotella, Pseudomonas, Sphingomonas Acinetobacter or Cutibacterium" where if the genus of the second bacterial strain is the same as that of the bacterial strain, the species is not identical; and where the genus and species are identical, the strains are not the same.

Preferably the dysbiotic condition includes at least one of acne, dandruff, dry skin, aging skin, pigmented skin or inflammation. More preferably it is acne.

Further preferably, when said dysbiotic condition is acne, said bacterial strain is from the genus Staphylococcus, Streptococcus, Microbacterium, Methyloversatilis, Deinococcus, Moraxella, or Acinetobacter.

Further preferably, the bacterial strain from the genus Staphylococcus is Staphylococcus hominis or Staphylococcus epidermidis.

The invention will now be explained with the help of non-limiting examples.

### Examples

### Example 1:

The procedure that was followed is disclosed hereinafter.

Facial buffer scrub samples (around 4 ml) were taken from 35 acne subjects and 32 non-acne subjects using a cylinder with diameter of 18 mm. Buffer samples were stored at -20°C before analysis. Microbes in the buffer scrub samples were pelleted by centrifugation (10 minutes/13,000 rpm, Eppendorf 5810R) at 4°C. The pellets were resuspended in 180 µl fresh enzymatic lysis buffer (20 mg/ml lysozyme (Sigma L6876) in 20 mM Tris·Cl, pH 8.0, 2 mM sodium EDTA and 1.2% Triton^{®} X-100). The mixtures were incubated at 37°C for 30 minutes, followed by addition of 25 µl proteinase K and 200 µl Buffer AL (without ethanol). It was further incubated at 56°C for 30 minutes.

Thereafter, 100 µl acid-wash glass beads (Sigma, G8772) were added into the tube and homogenized by MP Biomedicals FastPrep-24 (5 m/s, 45s, twice). Then DNA was extracted from the samples using DNA extraction kit (Qiagen, DNeasy Blood & Tissue kit, 69506) following the manufacturer's instructions. The microbial DNAs were sequenced for the variable region V1-V3 of the 16S rRNA gene for bacterial classification. First, the 16S V1-V3 gene was amplified using a primer set (forward: AGAGTTTGATYMTGGCTCAG, reverse: ATTACCGCGGCTGCTGG) recommended by sequencing company. 50 µl PCR reaction system consists of 1 µl (10 µM) of each forward and reverse primer, 5 µl of 10xPCR buffer, 2 µl of MgCl₂ (50 mM), 1.5 µl of dNTP mix (10 mM), 2 µl of DNA template, 0.2 µl of Platinum Taq DNA polymerase (Invitrogen) and 35.3 µl of molecular grade water (Sigma, W4502). For V1-V3 region, the samples were amplified using the following parameters: 94°C for 2 min, 20 cycles of: 94°C for 30 seconds, 65°C down 0.5°C per cycle for 1 minute; 10 cycles of: 94°C for 30s, 55°C for 1 minute; and 72°C for 5 minutes. To reduce the PCR amplification bias, each sample had three replicates. Two-step PCR method was used for library construction. A second round of PCR (8 cycles) was done by BGI by using fusion primers with dual index and adapters. The products were purified by Ampure beads. The quantity and quality of library were analysed by Bioanalyzer (Agilent Technologies). Only the qualified library was used for sequencing on Illumina Miseq PE300 platform.

Denoised sequencing data were clustered into Operational Taxonomic Units (OTUs) using Vsearch v1.9.6, with a cluster identity of 0.97 and a minimum cluster size of 10. OTUs were taxonomically classified against the SILVA, NCBI, RDP, DDBJ, Greengenes, CAMERA, EzBioCloud, and EMBL databases using a Lowest Common Ancestor (LCA) methodology.

The OTU table and associated representative sequences, selected as the most abundant sequence within the OTU cluster, were used as inputs for QIIME [46] version 1.9.1 (Quantitative Insights into Microbial Ecology), an open source software package for analysis of complex microbial communities. The output of QIIME was imported as a phyloseq object in R. For genus network, the OTU (operational taxonomic unit) table was merged to genus level before subsequent analysis. The prevalence threshold of OTUs for OTU network analysis was set as at least 50%.

Network analysis were conducted by Sparse InversE Covariance estimation for SPIEC-EASI version 0.1.2. This method estimates the inverse covariance matrix from sequencing data (Graphical Gaussian model). A lasso approach was applied to generate a sparse network. The neighbourhood and covariance method were chosen using Meinshausen and Buhlmann (MB) and the StARS (Stability Approach to Regularization Selection) selection method with a lambda max threshold of 0.01.

Cytoscape (version 3.5.1) were used to visualize OTU networks and calculate the network connectivity.

For the purpose of this experiment, *Staphylococcus* and *Cutibacterium* were the most abundant genera in healthy (non-dysbiotic condition) and acne (dysbiotic condition) microbiome.

To further explore interactions between the two genera, a subnetwork of OTUs was constructed from *Staphylococcus* and Cutibacterium.

As summarized in Table 1, connectivity of several OTUs were changed in the subnetwork between acne and non-acne microbiome.

OTU 722, the most abundant OTU of *Cutibacterium acnes,* was a hub node in acne. Its relative abundance did not transition significantly between acne and healthy conditions, but its connectivity with other bacteria was lower in healthy condition (degree = 4) but the connectivity transitioned to higher connectivity in acne condition (degree = 8).

We hypothesize that increased connectivity is a potential indication of pathogenicity of the strain in acne. In contrast, *Staphylococcus epidermidis* (OTU 24) is commonly regarded as commensal bacteria on skin. In acne condition, its relative abundance was increased, but its connectivity with other bacteria reduced (degree from 10 to 6), which suggests that S. *epidermidis* may have a different ecological role than *Cutibacterium acnes* in acne and healthy skin.

We also observed that *Staphylococcus hominis (S. hominis)* had higher connectivity with other bacteria in the *Staphylococcus* and *Cutibacterium* subnetwork.

OTU 2719 and OTU 227 of S. *hominis* were also highly connected in healthy skin condition (degree = 9 and 10 respectively), but their connectivity was reduced in acne or dysbiotic condition (degree = 5 and 2 respectively).

Sub-network analysis revealed possible ecological roles of a microbe, which may not always be represented in its relative abundance.

**Table 1**

| OTU | Species | Relative abundance (%) | | Degree | |
|---|---|---|---|---|---|
| | | Acne | Non-acne | Acne | Non-acne |
| 722 | C. acnes | 21.57 | 19.97 | 8 | 4 |
| 24 | S. epidermidis | 21.67* | 17.64 | 6 | 10 |
| 2719 | S. hominis | 0.10 | 0.2 | 5 | 9 |
| 227 | S. hominis | 0.44 | 1.24* | 2 | 10 |

| | | | | | |
|---|---|---|---|---|---|
| Note: In the table 1, * P<0.05 *S. hominis* is considered a minor species (mean relative abundance < 1%) in the microbiome of skin. Therefore, its functional relevance may be overlooked if the findings of traditional microbiome analysis is followed. However, based on the data indicated in Table 1, S. *hominis* could be a beneficial bacterium in acne skin. | | | | | |

### Example 2

By following the method disclosed in Example 1 with appropriate modifications, the relative abundance and degree of other bacteria potentially beneficial for treatment of acne were observed. The findings are summarised in Table 2.

**Table 2**

| OTU | Species | Relative abundance (%) | | Degree | |
|---|---|---|---|---|---|
| | | Acne | Non-acne | Acne | Non-acne |
| 264 | *Streptococcus sanguinis* | 0.010 | 0.017* | NA | 9 |
| 1915 | *Microbacterium arborescens* | 0.016 | 0.160* | NA | 8 |
| 2312 | *Methyloversatalis universalis* | 0.001 | 0.002* | NA | 5 |
| 2630 | *Deinococcus sp. X-121 sp* | 0..20 | 0.128* | NA | 8 |
| 1114 | *Moraxella osloensis* | 0.744 | 1.476* | 2 | 9 |
| 786 | *Acinetobacter johnsonii* | 0.006 | 0.023* | NA | 10 |

| | | | | | |
|---|---|---|---|---|---|
| * P<0.05 | | | | | |

## Claims

1. A method of determining a probiotic bacterial strain suitable for treating a human skin dysbiotic condition, comprising a step of doing network analysis by a computer to determine the connectivity of said bacterial strain with at least a second bacterial strain in a dysbiotic condition as well as a non-dysbiotic condition, wherein:
- said network is generated by co-occurrence analysis of abundance of said bacterial strain and said second bacterial strain by DNA sequencing by following 16s rRNA amplicon or whole genome sequencing method; and
- in said network, each node represents a species or an operation taxonomic unit (OTU), and each edge represents a connection between two such species or OTUs, said connection meaning a positive correlation or a negative correlation,
- wherein the network analysis is used to determine the degree of connectivity, meaning the number of edge connections, between said bacterial strain and other bacteria, in said dysbiotic condition as well as in said non-dysbiotic condition,
- and said bacterial strain is said to be suitable if the degree of connectivity of said bacterial strain is lower in said dysbiotic condition and higher in said non-dysbiotic condition.

2. A method as claimed in claim 1 wherein said difference in degree of connectivity is at least 40%.

3. A method as claimed in claim 1 or 2 wherein during said network analysis is set at the level of OTU (Operational Taxonomic Unit), ASV (Action Script Viewer), species or genus.

4. A method as claimed in any of claims 1 to 3 wherein said bacterial strain and said second bacterial strain are strains found on human skin.

5. A method as claimed in any of claims 1 to 4 wherein said bacterial strain is from at least one of the genus Staphylococcus, Streptococcus, Microbacterium, Methyloversatilis, Deinococcus, Moraxella or Acinetobacter.

6. A method as claimed in claim 5 wherein said second bacterial strain is from at least one of the genus Acidovorax, Actinomyces, Bacillus, Chryseobacterium, Corynebacterium, Fusobacterium, Staphylococcus, Streptococcus, Microbacterium, Methylobacterium, Methyloversatilis, Deinococcus, Micrococcus, Moraxella, Neisseria, Paracoccus, Prevotella, Pseudomonas, Sphingomonas Acinetobacter or Cutibacterium, where if the genus of the second bacterial strain is the same as that of the bacterial strain, the species is not identical; and where the genus and species are identical, the strains are not the same.

7. A method as claimed in any of claims 1 to 6 wherein said dysbiotic condition includes at least one of acne, dandruff, dry skin, aging skin, pigmented skin or inflammation.

8. A method as claimed in claim 7 wherein when said dysbiotic condition is acne, said bacterial strain is from the genus Staphylococcus, Streptococcus, Microbacterium, Methyloversatilis, Deinococcus, Moraxella or Acinetobacter.

9. A method as claimed in claim 8 wherein said bacterial strain from the genus Staphylococcus is Staphylococcus hominis or Staphylococcus epidermidis.

## Patentansprüche

1. Verfahren zur Bestimmung eines probiotischen Bakterienstamms, der zum Behandeln eines dysbiotischen Zustands menschlicher Haut geeignet ist, umfassend einen Schritt zum Durchführen einer Netzwerkanalyse durch einen Computer, um die Konnektivität des Bakterienstammes mit mindestens einem zweiten Bakterienstamm in einem dysbiotischen Zustand sowie in einem nicht-dysbiotischen Zustand zu bestimmen, wobei:
- das Netzwerk durch eine Co-Occurence-Analyse der Abundanz des Bakterienstamms und des zweiten Bakterienstamms durch DNA-Sequenzierung nach der 16s rRNA-Amplicon- oder Gesamtgenomsequenzierungsmethode erzeugt wird; und
- im Netzwerk jeder Knoten eine Art oder eine operative taxonomische Einheit (OTU) darstellt und wobei jede Kante eine Verbindung zwischen zwei derartigen Arten oder OTUs darstellt, wobei die Verbindung eine positive Korrelation oder eine negative Korrelation bedeutet,
- wobei die Netzwerkanalyse angewandt wird, um den Grad der Konnektivität zu bestimmen, was die Zahl der Kantenverbindungen zwischen dem Bakterienstamm und anderen Bakterien in dem dysbiotischen Zustand sowie in dem nicht-dysbiotischen Zustand bedeutet,
- und wobei der Bakterienstamm als geeignet gilt, wenn der Grad der Konnektivität des Bakterienstammes im dysbiotischen Zustand niedriger und im nicht-dysbiotischen Zustand höher ist.

2. Verfahren, wie in Anspruch 1 beansprucht, wobei der Unterschied im Grad der Konnektivität mindestens 40% beträgt.

3. Verfahren, wie in Anspruch 1 oder 2 beansprucht, wobei während der Netzwerkanalyse auf der Ebene OTU (Operational Taxonomic Unit), ASV (Action Script Viewer), Arten oder Gattung eingestellt wird.

4. Verfahren, wie in einem der Ansprüche 1 bis 3 beansprucht, wobei der Bakterienstamm und der zweite Bakterienstamm Stämme darstellen, die auf menschlicher Haut gefunden werden.

5. Verfahren, wie in einem der Ansprüche 1 bis 4 beansprucht, wobei der Bakterienstamm mindestens einer der Gattung Staphylococcus, Streptococcus, Microbacterium, Methyloversatilis, Deinococcus, Moraxella oder Acinetobacter ist.

6. Verfahren, wie in Anspruch 5 beansprucht, wobei der zweite Bakterienstamm mindestens einer der Gattung Acidovorax, Actinomyces, Bacillus, Chryseobacterium, Corynebacterium, Fusobacterium, Staphylococcus, Streptococcus, Microbacterium, Methylobacterium, Methyloversatilis, Deinococcus, Micrococcus, Moraxella, Neisseria, Paracoccus, Prevotella, Pseudomonas, Sphingomonas Acinetobacter oder Cutibacterium darstellt, wobei, wenn die Gattung des zweiten Bakterienstamms dieselbe wie die des Bakterienstamms ist, die Art nicht identisch ist; und wo die Gattung und die Art identisch sind, die Stämme nicht gleich sind.

7. Verfahren, wie in einem der Ansprüche 1 bis 6 beansprucht, wobei der dysbiotische Zustand mindestens einen von Akne, Schuppen, trockener Haut, alternder Haut, pigmentierter Haut oder Entzündung umfasst.

8. Verfahren, wie in Anspruch 7 beansprucht, wobei wenn der dysbiotische Zustand Akne ist, der Bakterienstamm von der Gattung Staphylococcus, Streptococcus, Microbacterium, Methyloversatilis, Deinococcus, Moraxella oder Acinetobacter ist.

9. Verfahren, wie in Anspruch 8 beansprucht, wobei der Bakterienstamm von der Gattung Staphylococcus Staphylococcus hominis oder Staphylococcus epidermis ist.

## Revendications

1. Procédé de détermination d'une souche bactérienne probiotique adaptée au traitement d'une affection dysbiotique cutanée humaine, comprenant une étape de réalisation d'une analyse de réseau par un ordinateur pour déterminer la connectivité de ladite souche bactérienne avec au moins une deuxième souche bactérienne dans une affection dysbiotique ainsi que dans une affection non dysbiotique, dans lequel:
- ledit réseau est généré par analyse de co-occurrence de l'abondance de ladite souche bactérienne et de ladite deuxième souche bactérienne par séquençage d'ADN selon le procédé de séquençage d'amplicon d'ARNr 16s ou du génome entier; et
- dans ledit réseau, chaque nœud représente une espèce ou une unité taxonomique opérationnelle (UTO), et chaque arête représente une connexion entre deux de telles espèces ou UTO, ladite connexion signifiant une corrélation positive ou une corrélation négative,
- dans lequel l'analyse de réseau est utilisée pour déterminer le degré de connectivité, c'est-à-dire le nombre de connexions d'arêtes, entre ladite souche bactérienne et d'autres bactéries, dans ladite affection dysbiotique ainsi que dans ladite affection non dysbiotique,
- et ladite souche bactérienne est dite être adaptée si le degré de connectivité de ladite souche bactérienne est plus faible dans ladite affection dysbiotique et plus élevé dans ladite affection non dysbiotique.

2. Procédé selon la revendication 1, dans lequel ladite différence de degré de connectivité est d'au moins 40 %.

3. Procédé selon la revendication 1 ou 2, dans lequel ladite analyse de réseau est définie au niveau de l'UTO (unité taxonomique opérationnelle), du VSA (visualiseur de scripts d'action), de l'espèce ou du genre.

4. Procédé selon l'une quelconque des revendications 1 à 3, dans lequel ladite souche bactérienne et ladite deuxième souche bactérienne sont des souches rencontrées sur la peau humaine.

5. Procédé selon l'une quelconque des revendications 1 à 4, dans lequel ladite souche bactérienne provient d'au moins l'un des genres Staphylococcus, Streptococcus, Microbacterium, Methyloversatilis, Deinococcus, Moraxella ou Acinetobacter.

6. Procédé selon la revendication 5, dans lequel ladite deuxième souche bactérienne provient d'au moins l'un des genres Acidovorax, Actinomyces, Bacillus, Chryseobacterium, Corynebacterium, Fusobacterium, Staphylococcus, Streptococcus, Microbacterium, Methylobacterium, Methyloversatilis, Deinococcus, Micrococcus, Moraxella, Neisseria, Paracoccus, Prevotella, Pseudomonas, Sphingomonas, Acinetobacter ou Cutibacterium, dans lequel si le genre de la deuxième souche bactérienne est le même que celui de la souche bactérienne, l'espèce n'est pas identique; et lorsque le genre et l'espèce sont identiques, les souches ne sont pas les mêmes.

7. Procédé selon l'une quelconque des revendications 1 à 6, dans lequel ladite affection dysbiotique inclut au moins l'une de l'acné, les pellicules, la peau sèche, la peau vieillissante, la peau pigmentée ou l'inflammation.

8. Procédé selon la revendication 7, dans lequel, lorsque ladite affection dysbiotique est l'acné, ladite souche bactérienne provient du genre Staphylococcus, Streptococcus, Microbacterium, Methyloversatilis, Deinococcus, Moraxella ou Acinetobacter.

9. Procédé selon la revendication 8, dans lequel ladite souche bactérienne provenant du genre Staphylococcus est Staphylococcus hominis ou Staphylococcus epidermidis.
